# EUROPEAN PATENT SPECIFICATION

(11) **EP 2 024 500 B1**
(45) Date of publication and mention of the grant of the patent: **01.02.2012**
(21) Application number: 07746891.6
(22) Date of filing: 13.06.2007
(51) Int. Cl.: C07K 14/82, G01N 33/48, G01N 33/50

(54) **Gene family (LBFL313) associated with pancreatic cancer**
Mit Pancreaskrebs assoziierte Genfamilie (LBFL313)
Famille de gène (lbfl313) associée au cancer du pancréas

(30) Priority: 14.06.2006 KR 20060053629
(43) Date of publication of application: 18.02.2009
(73) Proprietor: LG Life Sciences, Ltd., Seoul 150-721 (KR)
(72) Inventor: KOH, Sang-seok, Daejeon 305-308 (KR); SONG, Si-young, Seoul 102-140 (KR); KIM, Sun-a, Seoul 152-101 (KR); LEE, Yang-soon, Daejeon 305-390 (KR); JEON, Sun-bok, Daejeon 300-786 (KR); PARK, Eui-chul, Daejeon 305-728 (KR); KIM, Young-gun, Daejeon 302-740 (KR)
(74) Representative: Luderschmidt, Schüler & Partner
(86) International application number: PCT/KR2007/002848
(87) International publication number: WO 2007/145466

(56) References cited:
- EP-A- 1 526 381
- WO-A2-00/12708
- US-A- 4 962 048
- US-A1- 2006 088 876
- DATABASE EMBL [Online] 8 March 2005 (2005-03-08), "Homo sapiens zymogen granule protein 16 homolog B (rat), mRNA (cDNA clone MGC:9575 IMAGE:3879368), complete cds." XP002552477 retrieved from EBI accession no. EMBL:BC009722 Database accession no. BC009722
- GRUETZMANN ROBERT ET AL: "Microarray-based gene expression profiling in pancreatic ductal carcinoma: status quo and perspectives" INTERNATIONAL JOURNAL OF COLORECTAL DISEASE, vol. 19, no. 5, September 2004 (2004-09), pages 401-413, XP002552472 ISSN: 0179-1958
- JIN GANG ET AL: "Discovery and analysis of pancreatic adenocarcinoma genes using cDNA microarrays." WORLD JOURNAL OF GASTROENTEROLOGY : WJG 7 NOV 2005, vol. 11, no. 41, 7 November 2005 (2005-11-07), pages 6543-6548, XP002552473 ISSN: 1007-9327
- HUSTINX STEVEN R ET AL: "Differentially expressed genes in pancreatic ductal adenocarcinomas identified through serial analysis of gene expression." CANCER BIOLOGY & THERAPY DEC 2004, vol. 3, no. 12, December 2004 (2004-12), pages 1254-1261, XP002552474 ISSN: 1538-4047
- MULLINS JOHN J ET AL: "Identification of a human ortholog of the mouse Dcpp gene locus, encoding a novel member of the CSP-1/Dcpp salivary protein family." PHYSIOLOGICAL GENOMICS 13 DEC 2006, vol. 28, no. 1, 13 December 2006 (2006-12-13), pages 129-140, XP002552475 ISSN: 1531-2267
- KIM SUN A ET AL: "Pancreatic adenocarcinoma up-regulated factor (PAUF), a novel up-regulated secretory protein in pancreatic ductal adenocarcinoma." CANCER SCIENCE MAY 2009, vol. 100, no. 5, May 2009 (2009-05), pages 828-836, XP002552476 ISSN: 1349-7006

## Description

### [Technical Field]

The present invention relates to the changes in gene expression in pancreatic cancer tissues from pancreatic cancer patients. The invention specifically relates to a human gene which is differentially expressed in pancreatic cancer tissues compared to corresponding normal pancreatic tissues, and in other malignant neoplasms.

### [Background Art]

Pancreatic cancer, the fourth leading cause of cancer mortality in both man and woman, is a major health issue in the developed world and is associated with an exceedingly poor prognosis (Faint et al. (2004) Datamonitor DMHC2045; Garcea et al. (2005) Pancreatology 5:514-529; Kern et al. (2002) Cancer Biol Therapy 1:607-613; Laheru and Jaffee (2005) Nature Rev Cancer 5: 59-467; Li et al. (2004) Lancet 363:1049-1057). It has been estimated that about 30,000 Americans develop and die from this disease per year. In spite of aggressive surgical and medical management, the mean life expectancy is about 15~18 months for patients with local and regional disease, and 3~6 months for patients with metastatic disease. Close to 100% of patients with pancreatic cancer develop metastases and die because of the debilitating metabolic effects of their unrestrained growth and the overall five-year survival for the groups of patients who do not undergo resectional procedures is clearly less than five percent. It is particularly aggressive with non-specific initial symptoms and difficult early diagnosis. Early detection methods of pancreatic cancer are under development but do not yet exist in practice and conventional cancer therapies have little impact on prognosis or disease outcome. The poor prognosis of pancreatic cancer is attributable to its tendency for late presentation, aggressive local invasion, early metastasis and poor response to chemotherapy.

Like many other malignant disease, pancreatic cancer results from the accumulation of acquired mutations. Multiple genetic and epigenetic changes, including activation of protooncogenes, inactivation of tumor suppressor genes, and abnormalities of maintenance genes, are involved in the development, continued growth, and metastasis of pancreatic cancer. The accumulated mutations in such genes are believed to occur in a predictable time course during "PanINs" (Pancreatic Intraepithelial Neoplasia) stages (Hruban et al. (2000) Clin Cancer Res 6:2969-2972; Kern et al. (2002) Cancer Biol Therapy 1:607-613; Li et al. (2004) Lancet 363:1049-1057). Mutation of the K-ras occurs at half of the PanIN-1. The PanIN-2 stage is marked by additional changes and increase in the rate of K-ras mutations and by the appearance of numerous p16 abnormalities, and p53 protein overexpression, which may indicate the presence of p53 mutations, appears occasionally in the more advanced PanINs. Loss of tumor suppressor genes, TP53, DPC4, and BRCA2, appears to occur late in the development of pancreatic neoplasia, PanIN-3.

More than 85% of pancreatic ductal cancers have an activating point mutation the K-ras gene at pancreatic cancer development (Li et al. (2004) Lancet 363:1049-1057; Xiong (2004) Cancer Chem Pharm 54:S69-77). The K-ras mutation results in constitutive activation of an intracellular signaling pathway, Ras-Raf-MEK-ERK, leading to cellular proliferation and thus conferring transforming properties onto cells containing point mutations in this gene. Ras mutation is not associated with tumor stage or prognosis, indicating that the K-ras oncogene may be related to the initiation of carcinogenesis, but is not linked to malignant potential or promotion of human pancreatic cancer. One of the key downstream targets of the Ras family is phosphoinositol 3 kinase (PI3K). Activation of PI3K is implicated in pancreatic cancer resistance to apoptosis induced by chemotherapeutic or molecular targeting agents.

Inactivation of the p16 tumor suppressor gene appears to occur slightly later. The p16 gene is inactivated in virtually all ductal adenocarcinomas by mutation, homozygous deletion, or transcriptional silencing associated with promoter methylation (Kern et al. (2002) Cancer Biol Therapy 1:607-613; Maitra et al. (2006) Best Pract Res Clin Gastroenterol 20:211-226). The p16 protein regulates cell cycle through the p16/Rb pathway, therefore the genetic inactivation of the p16 gene means that a critical regulator of the cell cycle is lost in pancreatic cancer. Of interest, inherited mutations in the p16 gene are a cause of the Familial Atypical Multiple Mole Melanoma (FAMMM) syndrome, and patients with FAMMM have an increased risk of developing melanoma and pancreatic cancer.

The TP53 gene inactivation is almost always occurred by an intragenic mutation in one allele coupled with loss of the second allele (Maitra et al. (2006) Best Pract Res Clin Gastroenterol 20:211-226). The malfunction of p53 means that two critical controls of cell number, the G1/S cell cycle checkpoint and maintenance of G2/M arrest, are disregulated in the majority of pancreatic cancers.

The DPC4 gene, also known as SMAD4, is genetically inactivated in over half of pancreatic cancers, in 35% by homozygous deletion, and in 20% by an intragenic mutation coupled with loss of remaining allele (Maitra et al. (2006) Best Pract Res Clin Gastroenterol 20:211-226; Wilentz et al. (2000) Am J Pathol 156:37-43). However, genetic inactivation of DPC4 is only rarely appeared in other tumor types. The dpc4 protein plays a critical role in signaling and growth control through the TGF-B pathway.

BRCA2 gene, related to DNA repair, is only targeted in a small percentage (~10% ) of pancreatic cancer, but cause the familial aggregation of pancreatic cancer (Maitra et al. (2006) Best Pract Res Clin Gastroenterol 20:211-226; Murphy et al. (2002) Cancer Res 62:3789-3793). Carriers of a single base pair of the BRCA2 gene (called the 6174delT BRCA2 gene mutation) have a 10-fold increased risk of developing pancreatic cancer.

Nuclear factor κB (NF-κB), a transcription factor that predominantly exists as p65 (Re1A)/p50 heterodimer, is also regarded as one of the pancreatic cancer related genes (Garcea et al. (2005) Pancreatology 5:514-529; Xiong (2004) Cancer Chem Pharm 54:S69-77). RelA, the p65 subunit of NF-κB, is constitutively activated in around 67% of pancreatic adenocarcinomas, but not in healthy pancreatic tissues, and IκBα is overexpressed in human pancreatic tumor tissues and cell lines. The constitutive activation of RelA seems to be correlated with the upstream signaling pathway, such as Ras, in pancreatic tumor cells. It has been suggested that NF-κB play an important role in tumor resistance to apoptosis induced by cytotoxic agents in pancreatic cancer. Other results also say that the main mechanism by which NF-κB appears to promote pancreatic cell growth is via inhibition of apoptosis.

The entry (accession no. BC009722) from EMBL database is directed to a polynucleotide and a corresponding translated polypeptide sequence identical to SEQ ID NOs. 1 and 2. It does not disclose the use of said sequences for the diagnosis or treatment of pancreatic cancers.

EP 1 526 381 A discloses a diagnostic method to detect pancreatic adenocarcinoma using the DDEF2 protein, a development and differentiation enhancing factor. It does not disclose the use of the present sequences for the diagnosis or treatment of pancreatic cancers

There remains a need in the art for materials and methods permits a more accurate diagnosis of pancreatic adenocarcinoma. In addition there remains a need in the art for methods to treat and methods to identify agents that can effectively treat this disease. The present invention meets these and other needs.

### [Disclosure]

### [Technical Problem]

The present invention provides a material and method for an exactly diagnose pancreatic adenocarcinoma.

The present invention also provides material for treatment which effectively treats pancreatic adenocarcinoma.

### [Technical Solution]

The present invention is based on a new gene(hereinafter called as "LBFL313") that is differentially expressed in pancreatic adenocarcinoma tissues compared to normal pancreatic tissues. The invention provides a composition for diagnosing pancreatic cancer, which comprises (a) an isolated nucleic acid molecule comprising SEQ ID NO: 1, (b) an isolated nucleic acid molecule encoding SEQ ID NO: 2, (c) an isolated nucleic acid molecule exhibiting at least 95% nucleotide sequence identity over the entire contiguous sequence of SEQ ID NO: 1 and (d) an isolated nucleic acid molecule comprising the complement of a nucleic acid molecule of (a), (b) or (c).

The present invention further provides a composition for diagnosing pancreatic cancer, which comprises the nucleic acid molecules operably linked to one or more expression control elements, including vectors comprising the isolated nucleic acid molecules. The invention further provides a composition for diagnosing pancreatic cancer, which comprises host cells transformed to contain the nucleic acid molecules of the invention and methods for producing a protein comprising the step of culturing a host cell transformed with a nucleic acid molecule of the invention under conditions in which the protein is expressed.

The invention further provides a composition for diagnosing pancreatic cancer, which comprises an isolated polypeptide or protein comprising the amino acid sequence of SEQ ID NO: 2 or exhibiting at least 95% amino acid sequence identity with SEQ ID NO: 2.

### A. The Proteins Associated with Pancreatic Cancer

The present invention provides isolated proteins, allelic variants of the proteins, and conservative amino acid substitutions of the proteins. As used herein, the "protein" or "polypeptide" refers, in part, to a protein that has the human amino acid sequence depicted in SEQ ID NO: 2. The terms also refer to naturally occurring allelic variants and proteins that have a slightly different amino acid sequence than that specifically recited above. Allelic variants, though possessing a slightly different amino acid sequence than those recited above, will still have the same or similar biological functions associated with these proteins.

As used herein, the family of proteins related to the human amino acid sequence of SEQ ID NO: 2 refers to proteins that have been isolated from organisms in addition to humans. The methods used to identify and isolate other members of the family of proteins related to these proteins are described below.

The proteins of the present invention are preferably in isolated form. As used herein, a protein is said to be isolated when physical, mechanical or chemical methods are employed to remove the protein from cellular constituents that are normally associated with the protein. A skilled artisan can readily employ standard purification methods to obtain an isolated protein.

The proteins of the present invention further include insertion, deletion or conservative amino acid substitution variants of SEQ ID NO: 2. As used herein, a conservative variant refers to alterations in the amino acid sequence that do not adversely affect the biological functions of the protein. A substitution, insertion or deletion is said to adversely affect the protein when the altered sequence prevents or disrupts a biological function associated with the protein. For example, the overall charge, structure or hydrophobic/hydrophilic properties of the protein, in certain instances, may be altered without adversely affecting a biological activity. Accordingly, the amino acid sequence can be altered, for example to render the peptide more hydrophobic or hydrophilic, without adversely affecting the biological activities of the protein.

Ordinarily, the allelic variants, the conservative substitution variants, and the members of the protein family, will have an amino acid sequence having at least about 50%, 60%, 70% or 75% amino acid sequence identity with the sequence set forth in SEQ ID NO: 2, more preferably at least about 80-90%, even more preferably at least about 92-94%, and most preferably at least about 95%, 98% or 99% sequence identity. Identity or homology with respect to such sequences is defined herein as the percentage of amino acid residues in the candidate sequence that are identical with SEQ ID NO: 2, after aligning the sequences and introducing gaps, if necessary, to achieve the maximum percent homology, and not considering any conservative substitutions as part of the sequence identity (see section B for the relevant parameters). Fusion proteins, or N-terminal, C-terminal or internal extensions, deletions, or insertions into the peptide sequence shall not be construed as affecting homology.

Thus, the proteins disclosed herein include molecules having the amino acid sequence disclosed in SEQ ID NO: 2; fragments thereof having a consecutive sequence of at least about 3, 4, 5, 6, 10, 15, 20, 25, 30, 35 or more amino acid residues of these proteins; amino acid sequence variants wherein one or more amino acid residues has been inserted N- or C-terminal to, or within, the disclosed coding sequence; and amino acid sequence variants of the disclosed sequence, or their fragments as defined above, that have been substituted by at least one residue. Such fragments, also referred to as peptides or polypeptides, may contain antigenic regions, functional regions of the protein identified as regions of the amino acid sequence which correspond to known protein domains, as well as regions of pronounced hydrophilicity. The regions are all easily identifiable by using commonly available protein sequence analysis software such as MacVector (Oxford Molecular).

Contemplated variants further include those containing predetermined mutations by, e.g., homologous recombination, site-directed or PCR mutagenesis, and the corresponding proteins of other animal species, including but not limited to rabbit, mouse, rat, porcine, bovine, ovine, equine and non-human primate species, and the alleles or other naturally occurring variants of the family of proteins; and derivatives wherein the protein has been covalently modified by substitution, chemical, enzymatic, or other appropriate means with a moiety other than a naturally occurring amino acid (for example a detectable moiety such as an enzyme or radioisotope).

The present invention further provides compositions comprising a protein or polypeptide of the invention and a diluent. Suitable diluents can be aqueous or non-aqueous solvents or a combination thereof, and can comprise additional components, for example water-soluble salts or glycerol, that contribute to the stability, solubility, activity, and/or storage of the protein or polypeptide.

As described below, members of the family of proteins can be used: (1) to identify agents which modulate the level of or at least one activity of the protein, (2) to identify binding partners for the protein, (3) as an antigen to raise polyclonal or monoclonal antibodies, (4) as a therapeutic agent or target and (5) as a diagnostic agent or marker of pancreatic cancer and other hyperplastic diseases.

### B. Nucleic Acid Molecules

The present invention further provides nucleic acid molecules that encode the protein having SEQ ID NO: 2 and the related proteins herein described, preferably in isolated form. As used herein, "nucleic acid" is defined as RNA or DNA that encodes a protein or peptide as defined above, is complementary to a nucleic acid sequence encoding such peptides, hybridizes to the nucleic acid of SEQ ID NO: 1 and remains stably bound to it under appropriate stringency conditions, encodes a polypeptide sharing at least about 50%, 60%, 70% or 75%, preferably at least about 80-90%, more preferably at least about 92-94%, and most preferably at least about 95%, 98%, 99% or more identity with the peptide sequence of SEQ ID NO: 2 or exhibits at least 50%, 60%, 70% or 75%, preferably at least about 80-90%, more preferably at least about 92-94%, and even more preferably at least about 95%, 98%, 99% or more nucleotide sequence identity over the open reading frames of SEQ ID NO: 1.

The present invention discloses isolated nucleic acid molecules that specifically hybridize to the complement of SEQ ID NO: 1, particularly molecules that specifically hybridize over the open reading frames. Such molecules that specifically hybridize to the complement of SEQ ID NO: 1 typically do so under stringent hybridization conditions.

Specifically contemplated are genomic DNA, cDNA, mRNA and antisense molecules, as well as nucleic acids based on alternative backbones or including alternative bases, whether derived from natural sources or synthesized. Such hybridizing or complementary nucleic acids, however, are defined further as being novel and unobvious over any prior art nucleic acid including that which encodes, hybridizes under appropriate stringency conditions, or is complementary to nucleic acid encoding a protein according to the present invention.

Homology or identity at the nucleotide or amino acid sequence level is determined by BLAST (Basic Local Alignment Search Tool) analysis using the algorithm employed by the programs blastp, blastn, blastx, tblastn and tblastx (Altschul et al. (1997), Nucleic Acids Res. 25: 3389-3402, and Karlin et al. (1990), Proc. Natl. Acad. Sci. USA 87: 2264-2268, both fully incorporated by reference) which are tailored for sequence similarity searching. The approach used by the BLAST program is to first consider similar segments, with and without gaps, between a query sequence and a database sequence, then to evaluate the statistical significance of all matches that are identified and finally to summarize only those matches which satisfy a preselected threshold of significance. For a discussion of basic issues in similarity searching of sequence databases, see Altschul et al. (1994), Nat. Genet. 6: 119-129 which is fully incorporated by reference. The search parameters for histogram, descriptions, alignments, expect (i.e., the statistical significance threshold for reporting matches against database sequences), cutoff, matrix and filter (low complexity) are at the default settings. The default scoring matrix used by blastp, blastx, tblastn, and tblastx is the BLOSUM62 matrix (Henikoff et al. (1992), Proc. Natl. Acad. Sci. USA 89: 10915-10919, fully incorporated by reference), recommended for query sequences over 85 nucleotides or amino acids in length.

For blastn, the scoring matrix is set by the ratios of M (*i.e.*, the reward score for a pair of matching residues) to N (*i.e.*, the penalty score for mismatching residues), wherein the default values for M and N are 5 and -4, respectively. Four blastn parameters were adjusted as follows: Q=10 (gap creation penalty); R=10 (gap extension penalty); wink=1 (generates word hits at every wink^{th} position along the query); and gapw=16 (sets the window width within which gapped alignments are generated). The equivalent Blastp parameter settings were Q=9; R=2; wink=1; and gapw=32. A Bestfit comparison between sequences, available in the GCG package version 10.0, uses DNA parameters GAP=50 (gap creation penalty) and LEN=3 (gap extension penalty) and the equivalent settings in protein comparisons are GAP=8 and LEN=2.

"Stringent conditions" are those that (1) employ low ionic strength and high temperature for washing, for example, 0.015 M NaCl/0.0015 M sodium citrate/0.1 % SDS at 50 C, or (2) employ during hybridization a denaturing agent such as formamide, for example, 50% (vol/vol) formamide with 0.1% bovine serum albumin/0.1% Ficoll/0.1% polyvinylpyrrolidone/50 mM sodium phosphate buffer at pH 6.5 with 750 mM NaCl, 75 mM sodium citrate at 42 C. Another example is hybridization in 50% formamide, 5x SSC (0.75 M NaCl, 0.075 M sodium citrate), 50 mM sodium phosphate (pH 6.8), 0.1% sodium pyrophosphate, 5x Denhardt's solution, sonicated salmon sperm DNA (50 µg/ml), 0.1% SDS, and 10% dextran sulfate at 42 C, with washes at 42 C in 0.2× SSC and 0.1% SDS. A skilled artisan can readily determine and vary the stringency conditions appropriately to obtain a clear and detectable hybridization signal. Preferred molecules are those that hybridize under the above conditions to the complement of SEQ ID NO: 1 and which encode a functional or full-length protein. Even more preferred hybridizing molecules are those that hybridize under the above conditions to the complement strand of the open reading frame of SEQ ID NO: 1.

As used herein, a nucleic acid molecule is said to be "isolated" when the nucleic acid molecule is substantially separated from contaminant nucleic acid molecules encoding other polypeptides.

The present invention further provides fragments of the disclosed nucleic acid molecules. As used herein, a fragment of a nucleic acid molecule refers to a small portion of the coding or non-coding sequence. The size of the fragment will be determined by the intended use. For example, if the fragment is chosen so as to encode an active portion of the protein, the fragment will need to be large enough to encode the functional region(s) of the protein. For instance, fragments which encode peptides corresponding to predicted antigenic regions may be prepared. If the fragment is to be used as a nucleic acid probe or PCR primer, then the fragment length is chosen so as to obtain a relatively small number of false positives during probing/priming (see the discussion in Section G).

Fragments of the nucleic acid molecules of the present invention (i.e., synthetic oligonucleotides) that are used as probes or specific primers for the polymerase chain reaction (PCR), or to synthesize gene sequences encoding proteins of the invention, can easily be synthesized by chemical techniques, for example, the phosphoramidite method of Matteucci et al., ((1981) J. Am. Chem. Soc. 103: 3185-3191) or using automated synthesis methods. In addition, larger DNA segments can readily be prepared by well known methods, such as synthesis of a group of oligonucleotides that define various modular segments of the gene, followed by ligation of oligonucleotides to build the complete modified gene.

The nucleic acid molecules of the present invention may further be modified so as to contain a detectable label for diagnostic and probe purposes. A variety of such labels are known in the art and can readily be employed with the encoding molecules herein described. Suitable labels include, but are not limited to, biotin, radiolabeled or fluorescently labeled nucleotides and the like. A skilled artisan can readily employ any such label to obtain labeled variants of the nucleic acid molecules of the invention.

### C. Isolation of Other Related Nucleic Acid Molecules

As described above, the identification and characterization of the nucleic acid molecule having SEQ ID NO: 1 allows a skilled artisan to isolate nucleic acid molecules that encode other members of the protein family in addition to the sequences herein described. Further, the presently disclosed nucleic acid molecules allow a skilled artisan to isolate nucleic acid molecules that encode other members of the family of proteins in addition to the proteins having SEQ ID NO: 2.

For instance, a skilled artisan can readily use the amino acid sequence of SEQ ID NO: 2 to generate antibody probes to screen expression libraries prepared from appropriate cells. Typically, polyclonal antiserum from mammals such as rabbits immunized with the purified protein (as described below) or monoclonal antibodies can be used to probe a mammalian cDNA or genomic expression library, such as lambda gtll library, to obtain the appropriate coding sequence for other members of the protein family. The cloned cDNA sequence can be expressed as a fusion protein, expressed directly using its own control sequences, or expressed by constructions using control sequences appropriate to the particular host used for expression of the enzyme.

Alternatively, a portion of the coding sequence herein described can be synthesized and used as a probe to retrieve DNA encoding a member of the protein family from any mammalian organism. Oligomers containing approximately 18-20 nucleotides (encoding about a 6-7 amino acid stretch) are prepared and used to screen genomic DNA or cDNA libraries to obtain hybridization under stringent conditions or conditions of sufficient stringency to eliminate an undue level of false positives.

Additionally, pairs of oligonucleotide primers can be prepared for use in PCR to selectively clone an encoding nucleic acid molecule. A PCR denature/anneal/extend cycle for using such PCR primers is well known in the art and can readily be adapted for use in isolating other encoding nucleic acid molecules.

Nucleic acid molecules encoding other members of the protein family may also be identified in existing genomic or other sequence information using any available computational method, including but not limited to: PSI-BLAST (Altschul et al. (1997), Nucl. Acids Res. 25: 3389-3402); PHI-BLAST (Zhang et al. (1998), Nucl. Acids Res. 26: 3986-3990), 3D-PSSM (Kelly et al. (2000), J. Mol. Biol. 299: 499-520); and other computational analysis methods (Shi et al. (1999), Biochem. Biophys. Res. Commun. 262: 132-138 and Matsunami et. al. (2000), Nature 404: 601-604).

### D. rDNA molecules Containing a Nucleic Acid Molecule

The present invention further provides recombinant DNA molecules (rDNAs) that contain a coding sequence. As used herein, a rDNA molecule is a DNA molecule that has been subjected to molecular manipulation *in situ*. Methods for generating rDNA molecules are well known in the art, for example, see Sambrook et al., Molecular Cloning- A Laboratory, Manual, Third Ed., Cold Spring Harbor Laboratory Press, Cold Spring Harbor, NY, 2001. In the preferred rDNA molecules, a coding DNA sequence is operably linked to expression control sequences and/or vector sequences.

The choice of vector and/or expression control sequences to which one of the protein family encoding sequences of the present invention is operably linked depends directly, as is well known in the art, on the functional properties desired, *e.g.*, protein expression, and the host cell to be transformed. A vector contemplated by the present invention is at least capable of directing the replication or insertion into the host chromosome, and preferably also expression, of the structural gene included in the rDNA molecule.

Expression control elements that are used for regulating the expression of an operably linked protein encoding sequence are known in the art and include, but are not limited to, inducible promoters, constitutive promoters, secretion signals, and other regulatory elements. Preferably, the inducible promoter is readily controlled, such as being responsive to a nutrient in the host cell's medium.

In one embodiment, the vector containing a coding nucleic acid molecule will include a prokaryotic replicon, *i.e.*, a DNA sequence having the ability to direct autonomous replication and maintenance of the recombinant DNA molecule extrachromosomally in a prokaryotic host cell, such as a bacterial host cell, transformed therewith. Such replicons are well known in the art. In addition, vectors that include a prokaryotic replicon may also include a gene whose expression confers a detectable marker such as a drug resistance. Typical bacterial drug resistance genes are those that confer resistance to ampicillin, kanamycin, chloramphenicol or tetracycline.

Vectors that include a prokaryotic replicon can further include a prokaryotic or bacteriophage promoter capable of directing the expression (transcription and translation) of the coding gene sequences in a bacterial host cell, such as *E. coli*. A promoter is an expression control element formed by a DNA sequence that permits binding of RNA, polymerase and transcription to occur. Promoter sequences compatible with bacterial hosts are typically provided in plasmid vectors containing convenient restriction sites for insertion of a DNA segment of the present invention. Typical of such vector plasmids are pUC8, pUC9, pBR322 and pBR329 available from BioRad Laboratories, (Richmond, CA), pPL and pKK223 available from Pharmacia (Piscataway, NJ).

Expression vectors compatible with eukaryotic cells, preferably those compatible with vertebrate cells, can also be used to form rDNA molecules that contain a coding sequence. Eukaryotic cell expression vectors, including viral vectors, are well known in the art and are available from several commercial sources. Typically, such vectors are provided containing convenient restriction sites for insertion of the desired DNA segment. Typical of such vectors are pSVL and pKSV-10 (Pharmacia), pBPV-1/pML2d (International Biotechnologies, Inc.), pTDT1 (ATCC, #31255), the vector pCDM8 described herein, and the like eukaryotic expression vectors. Vectors may be modified to include tissue specific promoters if needed.

Eukaryotic cell expression vectors used to construct the rDNA molecules of the present invention may further include a selectable marker that is effective in a eukaryotic cell, preferably a drug resistance selection marker. A preferred drug resistance marker is the gene whose expression results in neomycin resistance, i.e., the neomycin phosphotransferase (*neo*) gene (Southern et al. (1982), J. Mol. Anal. Genet. 1:327-341). Alternatively, the selectable marker can be present on a separate plasmid, and the two vectors are introduced by co-transfection of the host cell, and selected by culturing in the appropriate drug for the selectable marker.

### E. Host Cells Containing an Exogenously Supplied Coding Nucleic Acid Molecule

The present invention further provides host cells transformed with a nucleic acid molecule that encodes a protein of the present invention. The host cell can be either prokaryotic or eukaryotic. Eukaryotic cells useful for expression of a protein of the invention are not limited, so long as the cell line is compatible with cell culture methods and compatible with the propagation of the expression vector and expression of the gene product. Preferred eukaryotic host cells include, but are not limited to, yeast, insect and mammalian cells, preferably vertebrate cells such as those from a mouse, rat, monkey or human cell line. Preferred eukaryotic host cells include Chinese hamster ovary (CHO) cells available from the ATCC as CCL61, NIH Swiss mouse embryo cells (NIH/3T3) available from the ATCC as CRL 1658, baby hamster kidney cells (BHK), and the like eukaryotic tissue culture cell lines.

Any prokaryotic host can be used to express a rDNA molecule encoding a protein of the invention. The preferred prokaryotic host is *E. coli*.

Transformation of appropriate cell hosts with a rDNA molecule of the present invention is accomplished by well known methods that typically depend on the type of vector used and host system employed. With regard to transformation of prokaryotic host cells, electroporation and salt treatment methods are typically employed (see, for example, Cohen et al. (1972), Proc. Natl. Acad. Sci. USA 69: 2110; and Sambrook *et al.*, *supra*). With regard to transformation of vertebrate cells with vectors containing rDNAs, electroporation, cationic lipid or salt treatment methods are typically employed, see, for example, Graham et al. (1973), Virol. 52: 456; Wigler et al. (1979), Proc. Natl. Acad. Sci. USA 76: 1373-1376.

Successfully transformed cells, i.e., cells that contain a rDNA molecule of the present invention, can be identified by well known techniques including the selection for a selectable marker. For example, cells resulting from the introduction of an rDNA of the present invention can be cloned to produce single colonies. Cells from those colonies can be harvested, lysed and their DNA content examined for the presence of the rDNA using a method such as that described by Southern, (1975) J. Mol. Biol. 98: 503 or Berent et al., (1985) Biotech. 3: 208, or the proteins produced from the cell assayed via an immunological method. The present inventors prepared a Escherichia coli DH5@/p313-JF3, and deposited them in Korean Collection for type Cultures of Korea Research Institute of Bioscience and Biotechnology on June 5, 2006 (Deposit No. KCTC 10954BP).

### F. Production of Recombinant Proteins using a rDNA Molecule

The present invention further discloses methods for producing a protein of the invention using nucleic acid molecules herein described. In general terms, the production of a recombinant form of a protein typically involves the following steps:

First, a nucleic acid molecule is obtained that encodes a protein of the invention, such as a nucleic acid molecule comprising, consisting essentially of or consisting of SEQ ID NO: 1, or nucleotides 53-643 or 53-640 of SEQ ID NO: 1. If the encoding sequence is uninterrupted by introns, as are these open-reading-frames, it is directly suitable for expression in any host.

The nucleic acid molecule is then preferably placed in operable linkage with suitable control sequences, as described above, to form an expression unit containing the protein open reading frame. The expression unit is used to transform a suitable host and the transformed host is cultured under conditions that allow the production of the recombinant protein. Optionally the recombinant protein is isolated from the medium or from the cells; recovery and purification of the protein may not be necessary in some instances where some impurities may be tolerated.

Each of the foregoing steps can be done in a variety of ways. For example, the desired coding sequences may be obtained from genomic fragments and used directly in appropriate hosts. The construction of expression vectors that are operable in a variety of hosts is accomplished using appropriate replicons and control sequences, as set forth above. The control sequences, expression vectors, and transformation methods are dependent on the type of host cell used to express the gene and were discussed in detail earlier. Suitable restriction sites can, if not normally available, be added to the ends of the coding sequence so as to provide an excisable gene to insert into these vectors. A skilled artisan can readily adapt any host/expression system known in the art for use with the nucleic acid molecules of the invention to produce recombinant protein.

### G. Rational Drug Design and Combinatorial Chemistry

The present invention further encompasses rational drug design and combinatorial chemistry. Those of skill will recognize appropriate methods to utilize and exploit aspects of the present invention in identifying compounds which can be developed for pancreatic cancer treatment. Rational drug design involving polypeptides requires identifying and defining a first peptide with which the designed drug is to interact, and using the first target peptide to define the requirements for a second peptide. With such requirements defined, one can find or prepare an appropriate peptide or non-peptide that meets all or substantially all of the defined requirements. Thus, one goal of rational drug design is to produce structural or functional analogs of biologically active polypeptides of interest or of small molecules with which they interact (e.g., agonists, antagonists, null compounds) in order to fashion drugs that are, for example, more or less potent forms of the ligand. (See, e.g., Hodgson (1991), Bio. Technology 9:19-21). Combinatorial chemistry is the science of synthesizing and testing compounds for bioactivity en masse, instead of one by one, the aim being to discover drugs and materials more quickly and inexpensively than was formerly possible. Rational drug design and combinatorial chemistry have become more intimately related in recent years due to the development of approaches in computer-aided protein modeling and drug discovery. (See e.g., US Pat. No. 4,908,773; 5,884,230; 5,873,052; 5,331,573; and 5,888,738).

The use of molecular modeling as a tool for rational drug design and combinatorial chemistry has dramatically increased due to the advent of computer graphics. Not only is it possible to view molecules on computer screens in three dimensions but it is also possible to examine the interactions of macromolecules such as enzymes and receptors and rationally designed derivative molecules to test. (See Boorman (1992), Chem. Eng. News 70:18-26). A vast amount of user-friendly software and hardware is now available and virtually all pharmaceutical companies have computer modeling groups devoted to rational drug design. Molecular Simulations Inc. (www.msi.com), for example, sells several sophisticated programs that allow a user to start from an amino acid sequence, build a two or three-dimensional model of the protein or polypeptide, compare it to other two and three-dimensional models, and analyze the interactions of compounds, drugs, and peptides with a three dimensional model in real time. Accordingly, in some embodiments of the invention, software is used to compare regions of the invention protein and molecules that interact therewith (collectively referred to as "binding partners" --e.g., anti-protein antibodies), and fragments or derivatives of these molecules with other molecules, such as peptides, peptidomimetics, and chemicals, so that therapeutic interactions can be predicted and designed. (See Schneider (1998), Genetic Engineering News December: page 20; Tempczyk et al. (1997), Molecular Simulations Inc. Solutions April; and Butenhof (1998), Molecular Simulations Inc. Case Notes (August 1998) for a discussion of molecular modeling).

### H. Gene Therapy

In another embodiment, genetic therapy can be used as a means for modulating biological and pathologic processes associated with the protein's function and activity. This comprises inserting into a cancerous cell a gene construct encoding a protein comprising all or at least a portion of the sequences of SEQ ID NO: 2, or alternatively a gene construct comprising all or a portion of the non-coding region of SEQ ID NO: 1, operably linked to a promoter or enhancer element such that expression of said protein causes suppression of said cancer and wherein said promoter or enhancer element is a promoter or enhancer element modulating said gene construct.

In the constructs described, expression of said protein can be directed from any suitable promoter (e.g., the human cytomegalovirus (CMV), simian virus 40 (SV40), or metallothionein promoters), and regulated by any appropriate mammalian regulatory element. For example, if desired, enhancers known to preferentially direct gene expression in neural cells, T cells, or B cells may be used to direct the expression. The enhancers used could include, without limitation, those that are characterized as tissue or cell specific in their expression. Alternatively, if a genomic clone of LBFL313 is used as a therapeutic construct (for example, following its isolation by hybridization with the nucleic acid molecule of the invention described above), regulation may be mediated by the cognate regulatory sequences or, if desired, by regulatory sequences derived from a heterologous source, including any of the promoters or regulatory elements described above.

Insertion of the construct into a cancerous cell is accomplished *in vivo*, for example using a viral or plasmid vector. Such methods can also be applied to *in vitro* uses. Thus, the methods of the present invention are readily applicable to different forms of gene therapy, either where cells are genetically modified *ex vivo* and then administered to a host or where the gene modification is conducted *in vivo* using any of a number of suitable methods involving vectors especially suitable to such therapies.

Retroviral vectors, adenoviral vectors, adeno-associated viral vectors, or other viral vectors with the appropriate tropism for cells likely to be involved in cancer (for example, epithelial cells) may be used as a gene transfer delivery system for a therapeutic gene construct. Numerous vectors useful for this purpose are generally known (Cozzi PJ, et al., (2002) Prostate, 53(2):95-100; Bitzer M, Lauer U., (2002) Dtsch Med Wochenschr. 127(31-32):1623-1624; Mezzina and Danos (2002), Trends Genet. 8:241-256; Loser et al. (2002) Curr. Gene Ther. 2:161-171; Pfeifer and Verma (2001), Annu. Rev. Genomics Hum. Genet. 2:177-211). Retroviral vectors are particularly well developed and have been used in clinical settings (Anderson et al. (1995), U.S. Patent No. 5,399,346). Non-viral approaches may also be employed for the introduction of therapeutic DNA into cells otherwise predicted to undergo cancer (Jeschke et al. (20002) Curr. Gene Ther. 1:267-278; Wu et al. (1988), J. Biol. Chem. 263:14621-14624; Wu et al. (1989), J. Biol. Chem. 264:16985-16987). For example, a gene may be introduced into a neuron or a T cell by lipofection, asialorosonucoid polylysine conjugation, or, less preferably, microinjection under surgical conditions.

For any of the methods of application described above, the therapeutic nucleic acid construct is preferably applied to the site of the cancer event (for example, by injection). However, it may also be applied to tissue in the vicinity of the cancer event or to a blood vessel supplying the cells predicted to undergo cancer.

### I. Diagnostic Methods

As the genes and proteins of the invention are differentially expressed in pancreatic cancer tissues compared to non-cancerous pancreatic tissues, the genes and proteins of the invention may be used to diagnose or monitor pancreatic cancer, to track disease progression, or to differentiate pancreatic tissue from non-cancerous pancreatic tissue samples. One means of diagnosing cancer using the nucleic acid molecules or proteins of the invention involves obtaining tissue from living subjects.

Assays to detect nucleic acid or protein molecules of the invention may be in any available format. Typical assays for nucleic acid molecules include hybridization or PCR based formats. Typical assays for the detection of proteins, polypeptides or peptides of the invention include the use of antibody probes in any available format such as *in situ* binding assays, etc. (see Harlow & Lane, Antibodies - A Laboratory Manual, Cold Spring Harbor Laboratory Press, Cold Spring Harbor, NY, 1988). In preferred embodiments, assays are carried-out with appropriate controls.

Generally, the diagnostics of the invention can be classified according to whether the embodiment is a nucleic acid or protein-based assay. Some diagnostic assays detect mutations or polymorphisms in the invention nucleic acids or proteins, which contribute to cancerous aberrations. Other diagnostic assays identify and distinguish defects in protein activity by detecting a level of invention RNA or protein in a tested organism that resembles the level of invention RNA or protein in a organism suffering from a disease, such as cancer, or by detecting a level of RNA or protein in a tested organism that is different than an organism not suffering from a disease.

Additionally, the manufacture of kits that incorporate the reagents and methods described in the following embodiments so as to allow for the rapid detection and identification of aberrations in protein activity or level are contemplated. The diagnostic kits can include a nucleic acid probe or an antibody or combinations thereof, which specifically detect a mutant form of the invention protein or a nucleic acid probe or an antibody or combinations thereof, which can be used to determine the level of RNA or protein expression of one or more invention protein. The detection component of these kits will typically be supplied in combination with one or more of the following reagents. A support capable of absorbing or otherwise binding DNA, RNA, or protein will often be supplied. Available supports include membranes of nitrocellulose, nylon or derivatized nylon that can be characterized by bearing an array of positively charged substituents. One or more restriction enzymes, control reagents, buffers, amplification enzymes, and non-human polynucleotides like calf-thymus or salmon-sperm DNA can be supplied in these kits.

Useful nucleic acid-based diagnostic techniques include, but are not limited to, direct DNA sequencing, gradient gel electrophoresis, Southern Blot analysis, single-stranded confirmation analysis (SSCA), RNAse protection assay, dot blot analysis, nucleic acid amplification, allele-specific PCR and combinations of these approaches. The starting point for these analyses is isolated or purified nucleic acid from a biological sample. It is contemplated that tissue biopsies would provide a good sample source. The nucleic acid is extracted from the sample and can be amplified by a DNA amplification technique such as the Polymerase Chain Reaction (PCR) using primers. Those of skill in the art will readily recognize methods available for confirming the presence of polymorphisms. In addition, any addressable array technology known in the art can be employed with this aspect of the invention. One particular embodiment of polynucleotide arrays is known as Genechips^{™}, and has been generally described in US Patent 5,143,854; PCT publications WO 90/15070 and 92/10092.

A wide variety of labels and conjugation techniques are known by those skilled in the art and can be used in various nucleic acid assays. There are several ways to produce labeled nucleic acids for hybridization or PCR including, but not limited to, oligolabeling, nick translation, end-labeling, or PCR amplification using a labeled nucleotide. Alternatively, a nucleic acid encoding an invention protein can be cloned into a vector for the production of an mRNA probe. Such vectors are known in the art, are commercially available, and can be used to synthesize RNA probes *in vitro* by addition of an appropriate RNA polymerase such as T7, T3 or Sup6 and labeled nucleotides. A number of companies such as Pharmacia Biotech (Piscataway, NJ), Promega (Madison, WI), and U.S. Biochemical Corp (Cleveland, OH) supply commercial kits and protocols for these procedures. Suitable reporter molecules or labels include those radionuclides, enzymes, fluorescent, chemiluminescent, or chromogenic agents, as well as, substrates, cofactors, inhibitors, magnetic particles and the like.

In preferred protein-based diagnostic, antibodies of the invention are attached to a support in an ordered array wherein a plurality of antibodies are attached to distinct regions of the support that do not overlap with each other. Those of skill in the art will readily recognize available assays that are protein-based diagnostics. Proteins are obtained from biological samples and are labeled by conventional approaches (e.g., radioactivity, colorimetrically, or fluorescently). Employing labeled standards of a known concentration of mutant and/or wild-type invention protein, an investigator can accurately determine the concentration of the invention protein in a sample and from this information can assess the expression level of the particular form of the protein. Conventional methods in densitometry can also be used to more accurately determine the concentration or expression level of such protein. These approaches are also easily automated using technology known to those of skill in the art of high throughput diagnostic analysis. As detailed above, any addressable array technology known in the art can be employed with this aspect of the invention and display the protein arrays on the chips in an attempt to maximize antibody binding patterns and diagnostic information.

As discussed above, the presence or detection of a polymorphism in an invention gene or protein can provide a diagnosis of a cancer or similar malady in an organism. Additional embodiments include the preparation of diagnostic kits comprising detection components, such as antibodies, specific for a particular polymorphic variant of invention gene or protein. The detection component will typically be supplied in combination with one or more of the following reagents. A support capable of absorbing or otherwise binding RNA or protein will often be supplied. Available supports for this purpose include, but are not limited to, membranes of nitrocellulose, nylon or derivatized nylon that can be characterized by bearing an array of positively charged substituents, and Genechips^{™} or their equivalents. One or more enzymes, such as Reverse Transcriptase and/or Taq polymerase, can be furnished in the kit, as can dNTPs, buffers, or non-human polynucleotides like calf-thymus or salmon-sperm DNA. Results from the kit assays can be interpreted by a healthcare provider or a diagnostic laboratory. Alternatively, diagnostic kits are manufactured and sold to private individuals for self-diagnosis.

In addition to diagnosing disease according to the presence or absence of a polymorphism, some diseases involving cancer result from skewed levels of invention protein or gene in particular tissues or aberrant patterns of invention protein expression. By monitoring the level of expression in various tissues, for example, a diagnosis can be made or a disease state can be identified. Similarly, by determining ratios of the level of expression of various invention proteins in specific tissues (e.g., patterns of expression) a prognosis of health or disease can be made. The levels of invention protein expression in various tissues from healthy individuals, as well as, individuals suffering from cancers is determined. These values can be recorded in a database and can be compared to values obtained from tested individuals. Additionally, the ratios or patterns of expression in various tissues from both healthy and diseased individuals is recorded in a database. These analyses are referred to as "disease state profiles" and by comparing one disease state profile (e.g. from a healthy or diseased individual) to a disease state profile from a tested individual, a clinician can rapidly diagnose the presence or absence of disease.
The nucleic acid and protein-based diagnostic techniques described above can be used to detect the level or amount or ratio of expression of invention genes or proteins in a tissue. Through quantitative Northern hybridizations, *in situ* analysis, immunohistochemistry, ELISA, genechip array technology, PCR, and Western blots, for example, the amount or level of expression of RNA or protein for a particular invention protein (wild-type or mutant) can be rapidly determined and from this information ratios of expression can be ascertained. Alternatively, the invention proteins to be analyzed can be family members that are currently unknown but which are identified based on their possession of one or more of the homology regions described above.

### [Description of Drawings]

Figure 1 Figure 1 shows the predicted signal sequence for secretion of LBFL313 (SEQ ID NO: 2). The analysis has been done using SignalIP 3.0 Server (www.cbs.dtu.dk/services/SignalIP/).
Figure 2 Figure 2 is result of Western analysis showing that LBFL313 is detected in culture supernatant of cells.
Figure 3 Figure 3 shows the effects of LBFL313 overexpression in CHO cells on cell proliferation (Panel A), motility (Panel B), and invasiveness (Panel C).
Figure 4 Figure 4 shows the effects of LBFL313 overexpression in nude mice on tumorigenesis (Panel A) and microvessel formation (Panel B).
Figure 5 Figure 5 shows representative results of immunohistochemical analysis of LBFL313 expression using pancreatic biopsy samples and anti-LBFL313 antibody.
Figure 6 Figure 6 depicts graphs showing the effects of polyclonal anti-LBFL313 antibody on invasiveness of pancreatic cancer cell lines.

### [Best Mode]

Without further description, it is believed that one of ordinary skill in the art can, using the preceding description and the following illustrative examples, make and utilize the compounds of the present invention and practice the claimed methods. The following working examples therefore, specifically point out preferred embodiments of the present invention, and are not to be construed as limiting in any way the remainder of the disclosure.

### EXAMPLES

### Example 1

### Identification of Differentially Expressed mRNA in Pancreatic Adenocarcinoma

Patient tissue samples were derived from Korean patients and classified into two groups. One group of consisted of patients who had been diagnosed with pancreatic adenocarcinoma. The patients in this group, six men and three women, ranged in age from 51-70. The second group of patients had been diagnosed with normal pancreas. In this group of three men, the patients ranged in age from 63-66. Histological analysis of each of the tissue samples was performed and samples were segregated into either non-cancerous or cancerous categories.

With minor modifications, the sample preparation protocol followed the Affymetrix GeneChip Expression Analysis Manual. Frozen tissue was first ground to powder using the Spex Certiprep 6800 Freezer Mill. Total RNA was then extracted using Trizol (Life Technologies). Next, mRNA was isolated using the Oligotex mRNA Midi kit (Qiagen). Using 1-5 mg of RNA, double stranded cDNA was created using the SuperScript Choice system (Gibco-BRL). First strand cDNA synthesis was primed with a T7-(dT24) oligonucleotide. The cDNA was then phenol-chloroform extracted and ethanol precipitated to a final concentration of 1 mg/ml.

From 2 mg of cDNA, cRNA was synthesized according to standard procedures. To biotin label the cRNA, nucleotides Bio-11-CTP and Bio-16-UTP (Enzo Diagnostics) were added to the reaction. After a 37°C incubation for six hours, the labeled cRNA was cleaned up according to the Rneasy Mini kit protocol (Qiagen). The cRNA was then fragmented (5' fragmentation buffer: 200 mM Tris-Acetate (pH 8.1), 500 mM KOAc, 150 mM MgOAc) for thirty-five minutes at 94°C.

Fifty five mg of fragmented cRNA was hybridized on the Affymetrix Human Genome U133 set of arrays for twenty-four hours at 60 rpm in a 45°C hybridization oven. The chips were washed and stained with Streptavidin Phycoerythrin (SAPE) (Molecular Probes) in Affymetrix fluidics stations. To amplify staining, SAPE solution was added twice with an anti-streptavidin biotinylated antibody (Vector Laboratories) staining step in between. Hybridization to the probe arrays was detected by fluorometric scanning (Hewlett Packard Gene Array Scanner). Following hybridization and scanning, the microarray images were analyzed for quality control, looking for major chip defects or abnormalities in hybridization signal. After all chips passed QC, Affymetrix Microarray Suite (v5.0), and LIMS (v3.0).

Differential expression of genes between the cancerous and non-cancerous pancreatic samples was determined by using Affymetrix human GeneChip sets U133, with the following statistical methods. (1) For each gene, signal values for U133 were determined by Affymetrix Microarray Suite (v5.0), which also made "Absent" (=not detected), "Present" (=detected) or "Marginal" (=not clearly Absent or Present) calls for each GeneChip element. (2) Using the criteria of at least 40% present call in cancerous pancreatic sample groups, a gene set was selected for further analysis. (3) All signal values were transformed to a logarithmic scale. (4) The Analysis of Variance (ANOVA) method was used for data analysis (Steel et al., Principles and Procedures of Statistics: A Biometrical Approach, Third Ed., McGraw-Hill, 1997).

Analysis of the chip data showed that the expression of the marker LBFL313 was significantly up-regulated (11.13-fold, p = 0) in pancreatic adenocarcinoma samples compared to samples from norman pancreatic tissue. These data indicate that up-regulation of LBFL313 may be diagnostic for pancreatic cancer.

The expression level of LBFL313 (SEQ ID NO: 1) can be measured by chip sequence fragment no. 228058_at on Affymetrix GeneChip U133. Through combined mining above data with the GeneExpress Oncology Datasuite^{™} of Gene Logic, Inc. (Gaithersburg, MD), the expression levels of 228058_at in various malignant neoplasms, compared to normal control tissues, are shown in Table 1, where the fold-change and the direction of the change (up- or down-regulation) are also indicated. A fold-change greater than 1.5 was considered to be significant.

The GeneChip expression results, determined by sample binding to chip sequence fragment no. 228058_at, were validated by quantitative RT-PCR (Q-RT-PCR) using the Taqman® assay (Perkin-Elmer). PCR primers designed from the sequence information file of the specific Affymetrix fragment (228058_at) were used in the assay. The target gene in each RNA sample (ten ng of total RNA) was assayed relative to an exogenously spiked reference gene. For this purpose, the tetracycline resistance gene was used as the exogenously added spike. This approach provides the relative expression as measured by cycle threshold (Ct) value of the target mRNA relative to a constant amount of Tet spike Ct values. The sample panel included tissue RNAs that were analyzed on U133 GeneChips. In addition, several new samples that were not analyzed on the GeneChip were used for the expression validations by Q-RT-PCR. The Q-RT-PCR data confirms the up-regulation of LBFL313 observed in pancreatic adenocarcinoma compared to normal pancreatic biopsy samples.

**Table 1.**

| **Expression of LBFL313 in malignant neoplasms** | | | | |
|---|---|---|---|---|
| Tissue | Pathology/Morphology | Fold Change | Direction | p value |
| Breast | Infiltrating Lobular Carcinoma | 1.64 | UP | 0.19 |
| | Infiltrating duct & Lobular Carcinoma | 1.59 | UP | 0.06 |
| Cervix | Squamous Cell Carcinoma | 1.61 | UP | 0.38 |
| Colon | Mucinous Adenocarcinoma | 1.88 | UP | 0.05 |
| Duodenum | Adenocarcinoma | 1.69 | UP | 0.19 |
| Esophagus | Adenocarcinoma | 1.83 | UP | 0.15 |
| Liver | Hepatocellular Carcinoma | 1.52 | UP | 0.02 |
| Lung | Adenocarcinoma | 1.52 | UP | 0.21 |
| Ovary | Mucinous Cystadenoarcinoma | 9.65 | UP | 0 |
| | Serous Cystadenocarcinoma | 2.15 | UP | 0.13 |
| Pancreas | Adenocarcinoma | 8.62 | UP | 0 |
| Skin | Basal Cell Carcinoma | -3.01 | DOWN | 0.01 |
| | Malignant Melanoma | -6.61 | DOWN | 0 |
| | Squamous Cell Carcinoma | -3.82 | DOWN | 0.03 |
| Stomach | Signet Ring Cell Carcinoma | 2,40 | UP | 0.03 |

### Example 2

### Cloning of Full-Length Human cDNA (LBFL313) Corresponding to Differentially Expressed mRNA Species

The full length cDNA having SEQ ID NO: 1 was obtained by the oligo-pulling method. Briefly, a gene-specific oligo was designed based on the sequence of LBFL313. The oligo was labeled with biotin and used to hybridize with 2 µg of single strand plasmid DNA (cDNA recombinants) from a human palcenta library following the procedures of Sambrook et al. The hybridized cDNAs were separated by streptavidin-conjugated beads and eluted by heating. The eluted cDNA was converted to double strand plasmid DNA and used to transform *E. coli* cells (DH10B) and the longest cDNA was screened. After positive selection was confirmed by PCR using gene-specific primers, the cDNA clone was subjected to DNA sequencing.

The nucleotide sequence of the full-length human cDNAs corresponding to the differentially regulated mRNA detected above is set forth in SEQ ID NO: 1. The cDNA comprises 777 base pairs.

An open reading frame within the cDNA nucleotide sequence of SEQ ID NO: 1, at nucleotides 53-640 (53-643 including the stop codon), encodes a protein of 196 amino acids. The amino acid sequence corresponding to a predicted protein encoded by SEQ ID NO: 1 is set forth in SEQ ID NO: 2.

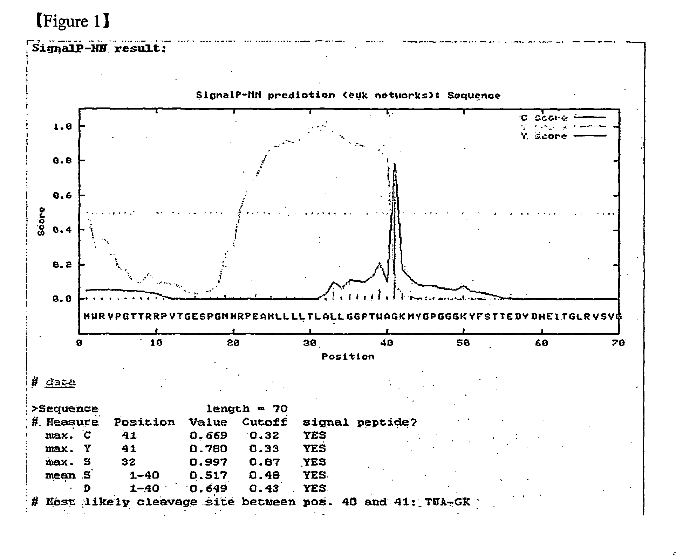

SEQ ID NO: 2 contains a Jacalin-like lectin domain: proteins containing this domain are lectins. It is found in 1 to 6 copies in these proteins. The domain is also found in the animal prostatic spermine-binding protein (Raval et al. (2004) Glycobiology 14:1247-1263). Figure 1 shows the result of signal sequence analysis by using SignalIP 3.0 Server (www.cbs.dtu.dk/services/SignalIP/). The potential signal sequence cleavage site for secretion is predicted between the amino acid position 40 (Ala) and 41 (Gly).

Analysis by Northern blot was performed to determine the size of the mRNA transcripts that correspond to LBFL313. A Northern blot containing total RNAs from various human tissues was used (Human 12-Lane MTN Blot, Clontech, Palo Alto, CA), and an EST containing 228058_at sequence was radioactively labeled by the random primer method and used to probe the blot. The blot was hybridized in 50% formamide, 5X SSPE, 0.1% SDS, 5X Denhart's solution, and 0.2 mg/ml herring sperm DNA at 42°C and washed with 0.2X SSC containing 0.1% SDS at room temperature. The Northern blot showed a single transcript for this gene, which is approximately 0.8 kb in size. This corresponds to the size of the LBFL313 clone (SEQ ID NO: 1).

### Example 3

### Production of LBFL313 Transfected Cell Lines

The coding region of LBFL313 was amplified by PCR using forward primer (5'-TTG GGATCCGTATAAAGGCGATGTGGAGG-3') incorporating the *Bam*HI site and reverse primer (5'-ACC ATC TAG AGC GAC CCA CGG GTG AGT-3') incorporating the *Xba*I site. PCR was performed using the TaqPlus precision DNA polymerase (Stratagene, CA) according to manufacturer's instruction. PCR amplification cycles involved initial denaturation at 94°C for 2 min, and 27 cycles ; 94°C for 30 sec, 50°C for 30 sec, 72 °C for 1 min, followed by a final extension at 72 °C for 10 min. The PCR product was cloned into the *Bam*HI and *Xba*I site of the mammalian expression vector pcDNA3.1-mycHis (Invitrogen). The cloned plasmid (pLFG250) was sequenced through the region of the cloning site to confirm its primary structure.

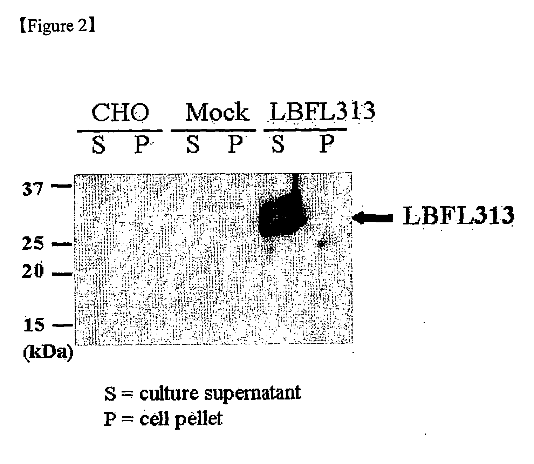

Subconfluent CHO cells were stably transfected with pLFG250 or with pcDNA3.1-mycHis vector alone using LipofectaminePLUS reagent (Invitrogen) according to manufacturer's instructions. After 24 h, transfected cells were cultured in Ham's F12 (Invitrogen) containing 10% FBS and 400 µg/ml G418 (Sigma) for selection. This selection medium was changed every 2 days, and after 10-12 days cloning rings were used to isolate positive clones. Cultures were further expanded and examined for LBFL313 expression. Cells were lysed in lysis buffer containing 50 mM HEPES (pH 7.2), 150 mM NaCl, 25mM beta-glyserophosphate, 25mM NaF, 5mM EGTA, 1mM EDTA, 1% NP-40, 1mM sodium orthovanadate, 0.1mM PMSF and protease inhibitor Protease Inhibitors cocktail (Leupeptin, Pepstatin, Aprotinin, and antipain each 5 µg/ml). Culture media were concentrated by 10k cutoff microcon (Amicon) make up to 15 µl volume. Proteins were reduced by incubation for 5 min at 95 °C in 4x SDS loading buffer. Polypeptides were resolved at 10 mA / gel on 12% SDS-PAGE gels and electrophoretically transfected to 0.45 µm Immobilon P-transfer membrane (Millipore) for 2 hr at 200 mA / gel at 4°C. Membranes were blocked for 1hr in TBST buffer (25 mM Tris, pH 7.5; 125 mM NaCl, and 0.1% (v/v) Tween-20) containing 5% (w/v) non-fat milk. Blots were then probed for overnight with anti-His HRP antibody (Santa Cruz). Immunoreactive material was then visualized by enhanced chemiluminescence (Elpis Biotech.) according to the manufacturer's instructions. As shown in Figure 2, LBFL313 protein was detected only in culture supernatant but not in cell extract. This confirms the prediction that LBFL313 encodes a secreted protein.

### Example 4

### Analysis of LBFL313 Overexpression on Cell Proliferation, Migration, and Invasion

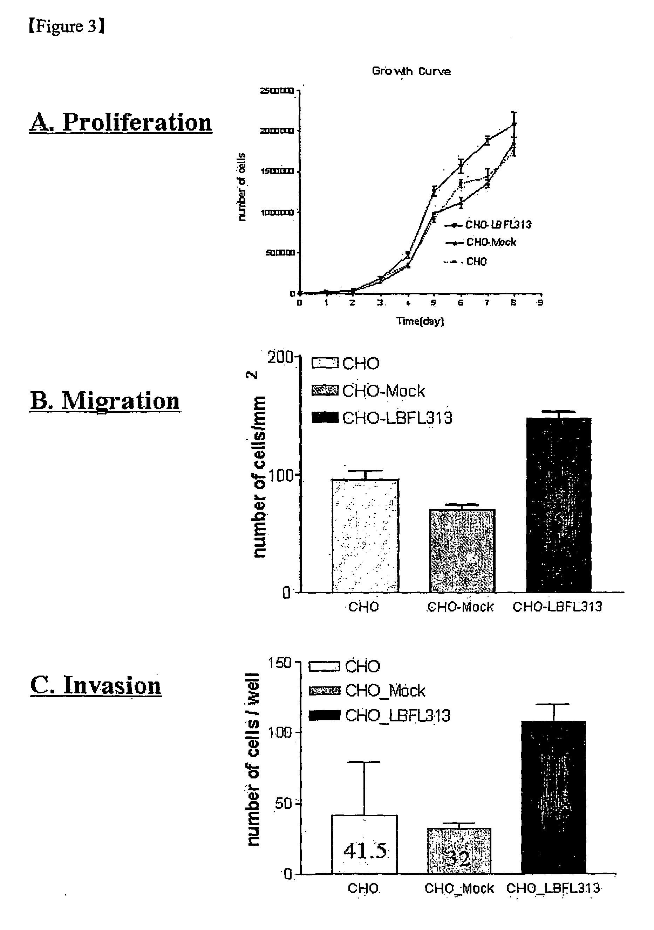

To determine the effect of LBFL313 overexpression on cell proliferation, growth rate was measured by cell counting. In 12 well plates, 4 x 10⁴ of cells were plated. The plates were incubated at 37°C and 5% CO₂ for 12 days and the number of cells was counted with hemocytometer every day. The result represent the mean values + standard deviation of triplication. As shown in Figure 3A, in CHO cells, overexpression of LBFL313 induced faster proliferation. Similar effect was observed with LBFL313-overexpressing PANC-1 pancreatic cancer cell line.

Migration and invasion was studied by Boyden Chamber assay. Both assays were done in a 48 well Boyden Chamber, Neuro Probe 48 well micro chamber (Neuroprobe). Cells were trypsinized and resuspended in trypsin inhibitor, Soybean trypsin inhibitor (Sigma) solution. The cells were pelleted and suspended to a final concentration of 2x10⁶ cells/ml in serum free media. The lower wells of the chamber were filled with 30 µl of standard media. The chamber was assembled using polycarbonate filters (polycarbonate, 8 µm diameter pore size, Neuroprobe). For cell invasion assays, 1mg/ml of Matrigel^{™} Basement Membrane Matrix (BD biosciences) was layered onto each filter (500 µg/filter) and air dried. Fifty µl sample of cell suspension (1x10⁵ cells/well) was added to the upper compartment. The chamber was incubated at 37 °C and 5% CO₂ and incubation time was varied depending on cell types to be analyzed: for 24hr in case of CHO migration assay, for 48hr in case of CHO invasion assay and for 18her in case of PANC-1 migration and invasion assay. At the end of incubation, the cells on the upper surface of the filters were mechanically removed. Filters were fixed in methanol and stained in Giemsa satin, modified solution (Sigma). The number of migrated cells per field (100x) were counted under the light microscope (Olympus). Each sample was assayed in triplicate. As shown in Figure 3B and Figure 3C, CHO cell motility and invasiveness were enhanced by overexpression of LBFL313. Similar effects were observed with LBFL313-overexpressing PANC-1 pancreatic cancer cell line.

### Example 5

### Effects of LBFL313 Overexpression on Tumorigenesis in Nude Mice

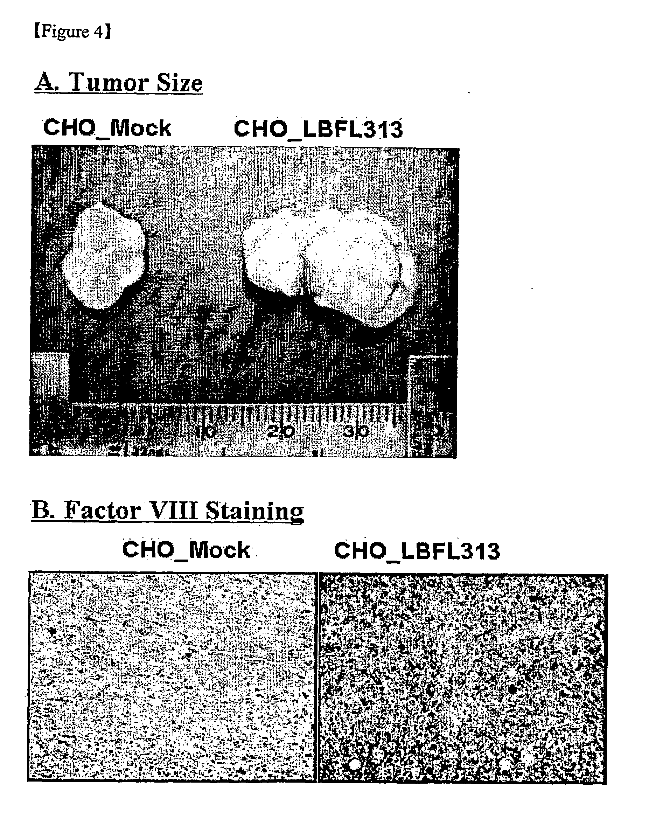

The biological effects of LBFL313 overexpression on tumor growth *in vivo* were determined. CHO cells were injected subcutaneously into the flanks of immunodeficient nude mice. Confluent CHO cells, untransfected or stably transfected with LBFL313 vector (pLFG250) or with vector alone, were trypsinized and resuspended in PBS at a density of 3.3 x 10⁷ cell/ml. Five million CHO cells of each type were injected subcutaneously into flank of five 8 weeks old female Balb/C (nu/nu) mice. In the process of mice growth, the lengths and widths of tumors were measured. Tumor volume was calculated with the formula Tumor Volume = (length) x (width)² / 2. After 37 days, the mice were sacrificed and tumors were analyzed. As shown in Figure 4A, the size of tumors generated by LBFL313-transfected cells was bigger more than 5-folds than that formed by mock control cells.

To determine the degree of tumor-induced angiogenesis, paraffin section of tumor xenografts were stained with anti-Factor VIII monoclonal antibody (Dako). Paraffin-embedded tissue sections (3-5 µm thickness) obtained from tumors of each group of mice were de-paraffinized in xylene and rehydrated in a graded ethanol series (100-90-80-70-50-30%) and the PCS washing. Endogenous peroxidase was blocked by immersing the slide in 0.3 % (v/v) hydrogen peroxide in methanol for 15 min at RT. After washing three times with PBS for 4 min each, the sections were blocked by soaking in 10 % (v/v) normal donkey serum in PBS for 1hr at RT. After washing three times with PBS for 4 min each, the blocked sections were incubated in anti-Factor VIII monoclonal antibody (von Willebrand factor, 1:50 dilution) (Dako) for 2 hr at RT. After 2hr, washing three times with PBS for 4 min each, the slide were incubated for 30 min with biotinylated Link universal at RT, washed three times in PBS for 4 min each, and incubated with streptoavidin-HRP conjugated (Dako) for 15 min at RT. After three times washing with PBS, the sections were incubated with chromogen and washed in distilled water. Factor VIII sections were not counterstained.

The number of microvessel was determined as described by Padro (Padro el al. (2000) Blood 95:2637-2644). Microvessel counting was simultaneously assessed by two independent experienced investigators using light microscopy. The investigators were not aware of the clinicopathologic finding. The entire section was systematically scanned, *ie*, field per field, at x100 magnification to find the areas showing the most intense vascularization. The magnification was then changed to x200 or to x400, and the investigators were allowed to reposition the slide until the highest number of microvessels was within the x400 field. This area was defined as a hot spot after achievement of a consensus between both investigators, thus reducing the inter-observer error of microvessel counting. In each hot spot, both investigators performed individual microvessel counting in a x400 field. As shown in Figure 4B, the results revealed that higher microvessel counts in the tumors from mice injected with LBFL313-transfected cells than with mock control cells. *In vivo* experiments implicate aggressive tumor formation and enhanced angiogenesis by LBFL313.

### Example 6

### Production of Polyclonal Anti-LBFL313 Antibody

LBFL313 full length cDNA was amplified by PCR using forward primer (5'-CTAAGGCCCAGCCGGCCGGGAAGATGTATGGCCCTGGA-3') and backward primer (5-'CATAGGCCCCACCGGCCGAGCGACCCACGGGTGAGTT-3'). PCR was performed using the TaqPlus precision DNA polymerase (Stratagene, CA) according to manufacturer's instruction. PCR amplification cycles involved initial denaturation at 94 °C for 5 min, and 30 cycles ; 94°C for 30 sec, 58°C for 30 sec, 72°C for 30 sec, followed by a final extension at 72°C for 10 min. The PCR product was visualized on a 1.5% TAE gel, and was gel purified, using the Zymoclean Gel DNA recovery kit (Zymo Research, CA) according to manufacturer's instruction. This purified DNA was inserted into SfiI site of pLFG106 vector to produce recombinant LBFL313-Fc fusion protein. pLFG106 is an expression vector containing signal sequence, Fc region of human IgG, and DHFR genes in the backbone of pcDNA3.1 (Invitrogen). PLFG106 also contains a thrombin recognition sequence between cloning site and Fc region. The LBFL313-Fc expression plasmid was stably transfected into DHFR-deficient CHO mutant cell lines using ExGen 500 reagents (Fermentas, Lithuania) according to manufacturer's instruction. Stably transfected cells were selected in nucleotide-free MEM-α (Invitrogen, CA) containing 800µg/ml of G418 (Invitrogen, CA) and 10% dialyzed FBS for 2 weeks. Stable transfectants were further adapted under a concentration of 100 nM methotrexate (Sigma, MO) for 2 weeks. Recombinant LBFL313-Fc fusion protein, secreted into the culture media, was identified by Western blot analysis and ELISA using anti-human Fc antibody (Sigma, MO).

Large-scale expression of recombinant LBFL313-Fc fusion protein was performed using Roller bottles(1750 cm2). Stable transfectants expressing recombinant LBFL313-Fc fusion proteins were grown in IMDM plus 5% FBS. The roller bottle culture was inoculated with 3 x 10⁸ cells and the device was rotated at 5 rpm in 37°C incubator. The cells were cultured for 5 days, and the medium was exchanged with serum-free medium every three days. Cell debris in harvested serum-free media was removed by the centrifugation at 6,000 rpm for 10 min. To purify the recombinant LBFL313-Fc fusion protein, cell-free medium was further processed by concentration using Concentrator, ProFlux M12 (Amicon) through a 10K MWCO Membrane, Pellicon 2 (Millipore). The concentrated medium was then passed through a 10-ml protein A agarose column (Pierce) pre-equilibrated in 20 mM Sodium phosphate, pH 8.0. Unbound protein was washed out of the column with 20 mM Sodium phosphate buffer. The column was then eluted with 0.1M Citrate, pH 3.0, collecting 4.5 ml fractions in tubes containing 500 µl 1M Tris-HCl, pH 9.0. Recombinant LBFL313-Fc fusion protein containing fractions were pooled and further purified with size-exclusion chromatography using sepharose 200HR resin (Amersham, IL).

The C-terminal Fc was cleaved from recombinant LBFL313-Fc fusion protein by Thrombin Cleavage Capture Kit (Novagen), and then removed by ImmunoPureR Immobilized Protein A (Pierce). Briefly, recombinant LBFL313-Fc fusion protein was incubated with biotinylated thrombin at 20 °C overnight. After proteolysis, biotinylated thrombin was removed by streptavidin agarose beads. The resulting solution, mixture of recombinant LBFL313 and Fc, was separated by passing twice through ImmunoPureR Immobilized Protein A column (Pierce). The purity of recombinant LBFL313 was checked on SDS-PAGE gels.

Purified recombinant LBFL313 was used to immunize two rabbits using standard techniques. Immunized serum was collected and then purified using ImmunoPureR (A Plus) IgG Purification Kit (Pierce) according to manufacturer's instruction. Further purification was performed using AminoLink^{R}(A Plus) Immobilization Kit (Pierce) linked with recombinant human Fc. The purified polyclonal antibody detects protein of approximately 21 kDa in Western blots of conditioned media obtained from pancreatic cancer cells expressing LBFL313. The specificity of the antibody was further demonstrated by enhanced detection of LBFL313 protein obtained from the conditioned media of LBFL313 transfected PANC-1 cells, while there was no enhancement from that of vector only transfected PANC-1 cells.

### Example 7

### Immunohistochemical Analysis of LBFL313 Expression

Tissue microarray slides were de-paraffinized in xylene and rehydrated in graded alcohol. Endogenous peroxidase activity was blocked with methanol containing 0.3% hydrogen peroxide at room temperature for 20 min. Microwave antigen retrieval was performed in citrate buffer (0.01M, pH 6.0) for 4 min. Then slides were incubated with 10% normal donkey serum solution for 1 hr to reduce background non-specific staining.

The primary antibody was polyclonal anti-LBFL313 antibody, at a dilution of 1:500. Blocked sections were incubated in primary antibody overnight at 4°C. The subsequent reaction was performed using an LSAB+ kit (DakoCytomation, Carpinteria, CA, USA) and the recommended procedure. Finally, the slides were incubated with 3-amino-9-ethyl carbazole (DakoCytomation, Carpinteria, CA, USA) and counterstained with Harris hematoxylin solution, modified (Sigma-Aldrich, Inc., St. Louis, MO, USA).

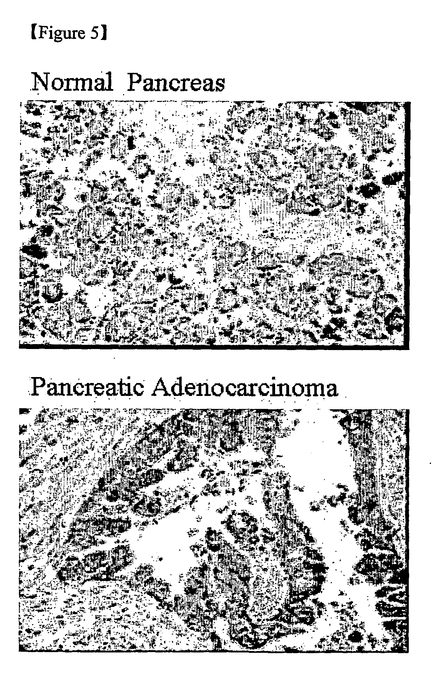

In all of cases, immunoreactivity was observed at the cytoplasm of tumor cells. Immunoreactivity was assessed by H-score method. The intensity of staining was scored as 0, 1, 2, and 3 corresponding to the presence of negative, weak, intermediate, and strong brown staining, respectively. The percentage of cells staining at different intensities was determined, and following formula was applied: H-score = (% of cells stained at intensity score 1) + 2 X (% of cells stained at intensity score 2) + 3 X (% of cells stained at intensity score 3). The H-scores of tumor tissue and adjacent non-tumor tissue were analyzed using the Wilcoxon signed rank test. As shown in Figure 5, tumor tissues had significantly higher LBFL313 expression than adjacent non-tumor tissues (14 out of 16 cases) (p<0.05).

### Example 8

### Effect of Anti-LBFL313 Antibody on Pancreatic Cancer Cell Invasiveness

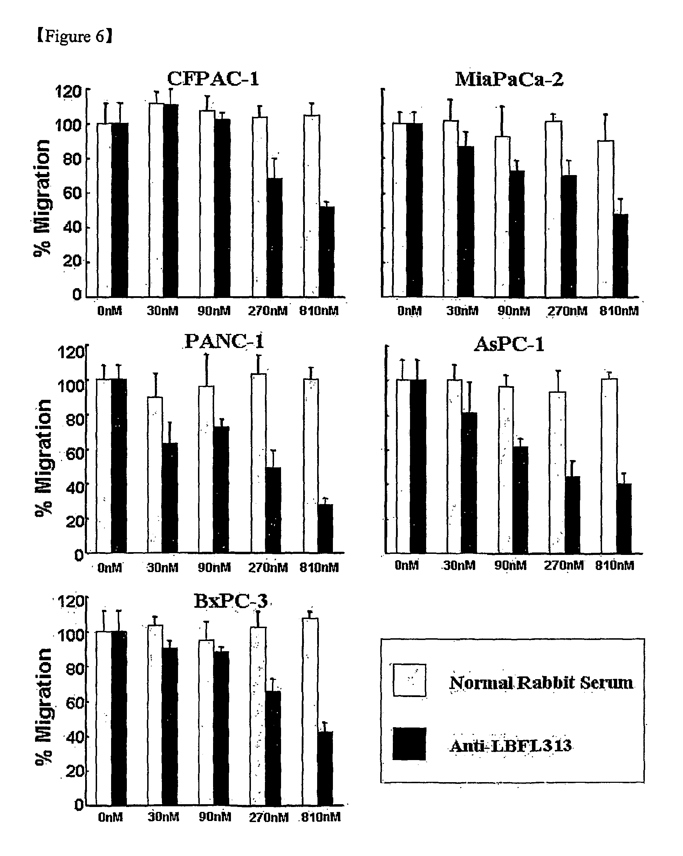

To determine the effect of anti-LBFL313 antibody on the invasion of human pancreatic cancer cell lines, Boyden Chamber assay was done in presence of anti-LBFL313 antibody or normal rabbit IgG. Briefly, five kinds of pancreatic cancer cell lines, CFPAC-1, MiaPaCa-2, PANC-1, AsPC-1 and BxPC-3, were trypsinized and resuspended in trypsin inhibitor (Sigma) solution. The cells were pelleted and suspended in serum free media in presence of PBS, anti-LBFL313 antibody, or normal rabbit IgG to a final concentration of 1~5x10⁶ cells/ml. The lower wells of the chamber were filled with 30 µl of standard media. The chamber was assembled using polycarbonate filters of 8 µm diameter pore size (Neuroprobe) coated on the upperside with 1 mg/ml of MatrigelTM Basement Membrane Matrix (BD biosciences). Fifty µl of cell suspension was added to the upper compartment. The chamber incubated at 37°C and 5% CO₂ for 24hr. At the end of incubation, the cells on the upper surface of the filters were mechanically removed. Filters were fixed in methanol and stained in Giemsa satin, modified solution (Sigma). The number of migrated cells per field (100x) was counted under the light microscope (Olympus). Each sample was assayed in triplicate. As shown in Figure 6, anti-LBFL313 antibody effectively inhibited invasiveness of human pancreatic cancer cell lines in a dose-dependent manner. Similar effects were observed in gastric cancer cell lines, AGS and N87.

### [Industrial Applicability]

Although the present invention has been described in detail with reference to examples above, it is understood that various modifications can be made . Accordingly, the invention is limited only by the following claims.

## Claims

1. A composition for diagnosing pancreatic cancers, which comprises an isolated nucleic acid molecule selected from the group consisting of: (a) an isolated nucleic acid molecule comprising SEQ ID NO: 1, (b) an isolated nucleic acid molecule encoding SEQ ID NO: 2, (c) an isolated nucleic acid molecule that encodes a protein that is expressed in cancer and that exhibits at least about 95% nucleotide sequence identity over the entire contiguous sequence of SEQ ID NO: 1, and (d) an isolated nucleic acid molecule comprising the complement of a nucleic acid molecule of (a), (b) or (c).

2. The composition according to claim 1, wherein the nucleic acid molecule comprises nucleotides 53-640 of SEQ ID NO: 1.

3. The composition according to claim 1, wherein the nucleic acid molecule comprises nucleotides 53-643 of SEQ ID NO: 1.

4. The composition according to any one of claims 1 to 3, wherein said nucleic acid molecule is operably linked to one or more expression control elements.

5. A composition for diagnosing pancreatic cancers, which comprises a vector comprising an isolated nucleic acid molecule selected from the group consisting of: (a) an isolated nucleic acid molecule comprising SEQ ID NO: 1, (b) an isolated nucleic acid molecule encoding SEQ ID NO: 2, (c) an isolated nucleic acid molecule that encodes a protein that is expressed in cancer and that exhibits at least about 95% nucleotide sequence identity over the entire contiguous sequence of SEQ ID NO: 1, and (d) an isolated nucleic acid molecule comprising the complement of a nucleic acid molecule of (a), (b) or (c).

6. A composition for diagnosing pancreatic cancers, which comprises a host cell transformed to contain an isolated nucleic acid molecule selected from the group consisting of: (a) an isolated nucleic acid molecule comprising SEQ ID NO: 1, (b) an isolated nucleic acid molecule encoding SEQ ID NO: 2, (c) an isolated nucleic acid molecule that encodes a protein that is expressed in cancer and that exhibits at least about 95% nucleotide sequence identity over the entire contiguous sequence of SEQ ID NO: 1, and (d) an isolated nucleic acid molecule comprising the complement of a nucleic acid molecule of (a), (b) or (c).

7. A composition for diagnosing pancreatic cancers, which comprises an isolated polypeptide or protein selected from the group consisting of protein comprising the amino acid sequence of SEQ ID NO: 2 and a protein having at least about 95% amino acid sequence identity with SEQ ID NO: 2.

8. A composition for diagnosing or treating pancreatic cancers, which comprises an isolated antibody or antigen-binding antibody fragment that binds to an isolated polypeptide selected from the group consisting of protein comprising the amino acid sequence of SEQ ID NO:2 and a protein having at least about 95% amino acid sequence identity SEQ ID NO:2.

9. A composition according to any one of claim 1 to 8, wherein the pancreatic cancer is pancreatic adenocarcinoma.

## Patentansprüche

1. Zusammensetzung zur Diagnose von Krebserkrankungen des Pankreas, welche ein isoliertes Nukleinsäuremolekül umfasst, ausgewählt aus der Gruppe bestehend aus: (a) einem isolierten Nukleinsäuremolekül, umfassend SEQ ID NO: 1, (b) einem isolierten Nukleinsäuremolekül, kodierend für SEQ ID NO: 2, (c) einem isolierten Nukleinsäuremolekül, das für ein Protein kodiert, das bei Krebs exprimiert wird, und das mindestens etwa 95 %ige Nukleotidsequenz-Identität über die gesamte zusammenhängende Sequenz der SEQ ID NO: 1 aufweist, und (d) ein isoliertes Nukleinsäuremolekül, umfassend das Komplement eines Nukleinsäuremoleküls nach (a), (b) oder (c).

2. Zusammensetzung nach Anspruch 1, wobei das Nukleinsäuremolekül die Nukleotide 53-640 der SEQ ID NO: 1 umfasst.

3. Zusammensetzung nach Anspruch 1, wobei das Nukleinsäuremolekül die Nukleotide 53-643 der SEQ ID NO: 1 umfasst.

4. Zusammensetzung nach einem der Ansprüche 1 bis 3, wobei das Nukleinsäuremolekül mit einem oder mehreren Expressionssteuerungselementen funktionell verbunden ist.

5. Zusammensetzung zur Diagnose von Krebserkrankungen des Pankreas, welche einen Vektor umfasst, umfassend ein isoliertes Nukleinsäuremolekül, ausgewählt aus der Gruppe bestehend aus: (a) einem isolierten Nukleinsäuremolekül, umfassend SEQ ID NO: 1, (b) einem isolierten Nukleinsäuremolekül, kodierend für SEQ ID NO: 2, (c) einem isolierten Nukleinsäuremolekül, das für ein Protein kodiert, das bei Krebs exprimiert wird, und das mindestens etwa 95 %ige Nukleotidsequenz-Identität über die gesamte zusammenhängende Sequenz der SEQ ID NO: 1 aufweist, und (d) ein isoliertes Nukleinsäuremolekül, umfassend das Komplement eines Nukleinsäuremoleküls nach (a), (b) oder (c).

6. Zusammensetzung zur Diagnose von Krebserkrankungen des Pankreas, welche eine Wirtszelle umfasst, die so umgewandelt ist, dass sie ein isoliertes Nukleinsäuremolekül enthält, ausgewählt aus der Gruppe bestehend aus: (a) einem isolierten Nukleinsäuremolekül, umfassend SEQ ID NO: 1, (b) einem isolierten Nukleinsäuremolekül, kodierend für SEQ ID NO: 2, (c) einem isolierten Nukleinsäuremolekül, das für ein Protein kodiert, das bei Krebs exprimiert wird, und das mindestens etwa 95 %ige Nukleotidsequenz-Identität über die gesamte zusammenhängende Sequenz der SEQ ID NO: 1 aufweist, und (d) ein isoliertes Nukleinsäuremolekül, umfassend das Komplement eines Nukleinsäuremoleküls nach (a), (b) oder (c).

7. Zusammensetzung zur Diagnose von Krebserkrankungen des Pankreas, welche ein isoliertes Polypeptid oder Protein umfasst, ausgewählt aus der Gruppe bestehend aus Protein, umfassend die Aminosäuresequenz der SEQ ID NO: 2 und einem Protein mit mindestens etwa 95 %iger Aminosäuresequenz-Identität mit SEQ ID NO: 2.

8. Zusammensetzung zur Diagnose oder Behandlung von Krebserkrankungen des Pankreas, welche einen isolierten Antikörper oder ein isoliertes Antigen-bindendes Antikörperfragment umfasst, das an ein isoliertes Polypeptid bindet, ausgewählt aus der Gruppe bestehend aus Protein, umfassend die Aminosäuresequenz der SEQ ID NO: 2 und einem Protein mit mindestens etwa 95 %iger Aminosäuresequenz-Identität mit SEQ ID NO: 2.

9. Zusammensetzung nach einem der Ansprüche 1 bis 8, wobei die Krebserkrankung des Pankreas Adenokarzinom des Pankreas ist.

## Revendications

1. Une composition servant à diagnostiquer les cancers pancréatiques, lesquels comprennent une molécule d'acide nucléique isolée, sélectionnée d'un groupe consistant de: (a) une molécule d'acide nucléique isolée comprenant le SEQ ID NO : 1, (b) une molécule d'acide nucléique isolée encodant le SEQ ID NO :2, (c) une molécule d'acide nucléique isolée qui encode une protéine exprimée dans un cancer et qui exhibe une identité nucléotidique séquentielle d'au moins 95% sur l'ensemble de la séquence contigüe de SEQ ID NO :1, et (d) une molécule d'acide nucléique isolée comprenant un complément d'une molécule d'acide nucléique de (a), (b) ou (c).

2. La composition selon la revendication 1, auquel la molécule d'acide nucléique comprend des nucléotides 53-640 de SEQ ID NO : 1.

3. La composition selon la revendication 1, auquel la molécule d'acide nucléique comprend des nucléotides 53-643 de SEQ ID NO : 1.

4. La composition selon n'importe laquelle revendications 1 à 3, auquel la molécule d'acide nucléique en question est opérationnellement liée à l'une ou plusieurs expressions d'éléments de contrôle.

5. Une composition pour le diagnostic de cancers pancréatiques, laquelle comporte un vecteur comportant une molécule d'acide nucléique isolée provenant du groupe consistant de: (a) une molécule d'acide nucléique isolée comportant le SEQ ID NO : 1, (b) une molécule d'acide nucléique isolée encodant le SEQ ID NO : 2, (c) une molécule d'acide nucléique isolée qui encode une protéine exprimée dans un cancer et exhibant une identité nucléotidique séquentielle d'au moins 95% sur l'ensemble contigüe de SEQ ID NO : 1, et (d) une molécule d'acide nucléique isolée comprenant le complément d'une molécule d'acide nucléique de (a), (b) ou (c).

6. Une composition pour le diagnostic de cancers pancréatiques, laquelle comporte une cellule hôte transformée afin de contenir une molécule d'acide nucléique isolée sélectionnée d'un groupe consistant de:
(a) une molécule d'acide nucléique isolée comportant le SEQ ID NO : 1,
(b) une molécule d'acide nucléique isolée encodant le SEQ ID NO : 2,
(c) une molécule d'acide nucléique isolée qui encode une protéine exprimée dans un cancer et exhibant une identité nucléotidique séquentielle d'au moins 95% sur l'ensemble contigüe de SEQ ID NO : 1, et (d) une molécule d'acide nucléique isolée comprenant le complément d'une molécule d'acide nucléique de (a), (b) ou (c).

7. Une composition pour le diagnostic de cancers pancréatiques, laquelle comporte une polypeptide isolée ou une protéine sélectionnée d'un groupe consistant de protéines comportant la séquence d'acide aminée SEQ ID NO: 2 et une protéine détenant une identité nucléotidique séquentielle d'au moins 95% avec le SEQ ID NO : 2.

8. Une composition pour le diagnostic ou le traitement de cancers pancréatiques, laquelle comporte un anticorps ou un antigène combinant un fragment d'anticorps, lié à une polypeptide isolée sélectionnée d'un groupe consistant de protéines recelant une séquence d'acide aminée de SEQ ID NO : 2 et une protéine détenant une identité d'acide aminée séquentielle d'au moins 95% avec le SEQ ID NO : 2.

9. Une composition selon une des revendications de 1 à 8, auquel le cancer pancréatique est un adénocarcinome pancréatique.


### SEQUENCE LISTING

<110> KOH, Sang Seok
   SONG, Si Young
   KIM, Sun-A
   LEE Yangsoon
   JEON, Sun Bok
   PARK, Euichul
   KIM, Young-Gun
<120> GENE FAMILY (LBFL313) ASSOCIATED WITH PANCREATIC CANCER
<130>
<160> 2
<170> FastSEQ for Windows Version 4.0
<210> 1
   <211> 777
   <212> DNA
   <213> Homo sapiens
<220>
   <221> CDS
   <222> (53)...(643)
   <223> Clone LBFL313
<400> 1
<210> 2
   <211> 196
   <212> PRT
   <213> Homo sapiens
<400> 2
   Met Trp Arg Val Pro Gly Thr Thr Arg Arg Pro Val Thr Gly Glu Ser
   Pro Gly Met His Arg Pro Glu Ala Met Leu Leu Leu Leu Thr Leu Ala
   Leu Leu Gly Gly Pro Thr Trp Ala Gly Lys Met Tyr Gly Pro Gly Gly
   Gly Lys Tyr Phe Ser Thr Thr Glu Asp Tyr Asp His Glu Ile Thr Gly
   Leu Arg Val Ser Val Gly Leu Leu Leu Val Lys Ser Val Gln Val Lys
   Leu Gly Asp Ser Trp Asp Val Lys Leu Gly Ala Leu Gly Gly Asn Thr
   Gln Glu Val Thr Leu Gln Pro Gly Glu Tyr Ile Thr Lys Val Phe Val
   Ala Phe Gln Ala Phe Leu Arg Gly Met Val Met Tyr Thr Ser Lys Asp
   Arg Tyr Phe Tyr Phe Gly Lys Leu Asp Gly Gln Ile Ser Ser Ala Tyr
   Pro Ser Gln Glu Gly Gln Val Leu Val Gly Ile Tyr Gly Gln Tyr Gln
   Leu Leu Gly Ile Lys Ser Ile Gly Phe Glu Trp Asn Tyr Pro Leu Glu
   Glu Pro Thr Thr Glu Pro Pro Val Asn Leu Thr Tyr Ser Ala Asn Ser
   Pro Val Gly Arg